(19) Europäisches Patentamt
European Patent Office
Office européen des brevets

(11) EP 4 389 214 A2

(12) # DEMANDE DE BREVET EUROPEEN

(43) Date de publication:
**26.06.2024 Bulletin 2024/26**

(21) Numéro de dépôt: **24174263.4**

(22) Date de dépôt: **28.09.2018**

(51) Classification Internationale des Brevets (IPC):
***A61P 17/16*** *(2006.01)*

(52) Classification Coopérative des Brevets (CPC):
**A61K 31/216; A61K 8/37; A61K 8/496; A61K 8/4966; A61K 9/0014; A61K 9/06; A61P 17/16; A61Q 17/04;** A61K 2800/522

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **05.10.2017 FR 1759351**

(62) Numéro(s) de document de la (des) demande(s) initiale(s) en application de l'article 76 CBE:
**18197698.6 / 3 466 419**

(71) Demandeur: **Thorel, Jean-Noël**
**75014 Paris (FR)**

(72) Inventeur: **Thorel, Jean-Noël**
**75014 Paris (FR)**

(74) Mandataire: **Cabinet Laurent & Charras**
**Le Contemporain**
**50 Chemin de la Bruyère**
**69574 Dardilly Cedex (FR)**

Remarques:
Cette demande a été déposée le 06-05-2024 comme demande divisionnaire de la demande mentionnée sous le code INID 62.

(54) **UTILISATION DU TRIMETHOXYBENZYL ACETYLSINAPATE, AVANTAGEUSEMENT EN COMBINAISON AVEC AU MOINS UN FILTRE SOLAIRE, POUR LA PROTECTION DE LA PEAU**

(57) La présente invention concerne l'utilisation du trimethoxybenzyl acetylsinapate, avantageusement en combinaison avec au moins un filtre solaire, pour la protection de la peau.

EP 4 389 214 A2

## Description

### Domaine de l'invention

La présente invention concerne notamment des compositions cosmétiques ou dermatologiques destinées à la protection des cellules de la peau et/ou des phanères contre le rayonnement solaire, en particulier le rayonnement ultraviolet.

### Etat de la technique

Le soleil émet plusieurs catégories de rayonnement dont une partie pénètre l'atmosphère, notamment les rayons ultraviolets (100 à 400 nm), la lumière visible (400 et 700 nm) et la lumière infrarouge (700 nm à 1 mm).

Le soleil a certains effets bénéfiques avérés chez l'homme, tels que son action antirachitique ou antidépressive. Les radiations lumineuses, notamment les ultraviolets de type A et B (notés UV-A et UV-B) émises par le soleil sont nécessaires au bon fonctionnement de l'organisme humain, mais elles provoquent également des effets délétères (photodermatoses et photo-cancérogenèse notamment).

Le rayonnement ultraviolet (UV) se compose lui-même de lumière de longueur d'onde comprise entre 100 nm et 400 nm, traditionnellement répartie en 3 sous-groupes : les UV-A (400-315 nm), les UV-B (315-280 nm) et les UV-C (280-100 nm). Les UV-C, de courte longueur d'onde, sont les UV les plus énergétiques et les plus nocifs, mais sont filtrés par la couche d'ozone de l'atmosphère et n'atteignent pas la surface de la Terre. Toutefois, en raison de la diminution de la couche d'ozone dans certaines régions du globe, la lumière solaire parvenant à la surface de la Terre a tendance à s'enrichir en rayonnements UV, notamment les UV-C (Lloyd *et al.,* 1993). La plupart des dégâts sur la peau restent malgré tout causés par les UV-A et les UV-B.

Les UV-B sont plus particulièrement à l'origine de la stimulation de la production du pigment naturel de la peau appelé mélanine, ce phénomène étant communément appelé le bronzage. Les UV-B stimulent également les cellules de la peau pour qu'elles produisent un épiderme plus épais. Ces réactions constituent un mécanisme de défense de l'organisme contre les rayons UV. Les UV-B sont plus énergétiques que les UV-A et sont responsables de l'érythème solaire (ou « coup de soleil »), qui manifeste son maximum d'intensité inflammatoire 24 heures après l'exposition. L'importante énergie des UV-B entraine également des désordres moléculaires, comme une altération de l'ADN et des dommages aux protéines (notamment leur carbonylation). A long terme, ces dégradations saturent et bloquent le système de réparation de 1'ADN nucléaire. Cela entraîne des mutations permanentes dans le génome des cellules atteintes, induisant l'apparition de cancers cutanés. On parle ainsi de toxicité directe des UV-B.

Les UV-A, qui ne présentent pas d'effets nocifs visibles à court terme, ont longtemps été considérés comme inoffensifs. Cependant, il est désormais largement reconnu que les UV-A pénètrent dans les couches profondes de la peau, et ont un effet sur le tissu conjonctif et les vaisseaux sanguins. Ces rayons sont notamment à l'origine du phénomène nommé héliodermie, c'est-à-dire le vieillissement actinique prématuré de la peau.

De surcroît, bien que les UV-B soient les principaux responsables des cancers cutanés, les UV-A ont également une contribution indirecte à ce type d'atteintes. En effet, les UV-A sont à l'origine de la production de radicaux libres, notamment les ROS (« *Reactive Oxygen Species* » ou espèces réactives de l'oxygène (ERO)). Ces molécules sont très instables et possèdent une demi-vie très courte, pouvant aller de la nanoseconde à la milliseconde. Les ROS sont capables d'endommager aussi bien les structures cellulaires (ADN, membranes, protéines intracellulaires), que les structures extracellulaires (composants de la matrice extracellulaire tels que les fibres de collagène). Les ROS exercent également une action nocive indirecte, en provoquant l'oxydation des lipides membranaires, ce qui induit la formation d'espèces réactives carbonylées, contribuant à amplifier les dégâts tissulaires et cellulaires causés. Les mêmes dommages sont également causés voire amplifiés par la pollution environnementale, qui génère aussi des radicaux libres responsables du vieillissement cutané.

Une préoccupation permanente est donc de combattre les effets nocifs des rayonnements UV, notamment UV-A et UV-B, en prenant en compte le type de peau (phototype) et les temps d'exposition au soleil.

Il existe deux façons de minimiser les dégâts causés par le soleil sur la peau : la protection externe et la protection interne.

La protection externe est réalisée en filtrant le rayonnement UV qui pénètre la peau ou en occultant les zones exposées au soleil. Bien que le recouvrement des zones exposées assure la meilleure photoprotection, elle n'est pas toujours possible ou pratique. Pour cette raison, des compositions solaires sont nécessaires afin d'assurer une photoprotection des zones exposées au rayonnement UV. Ces compositions se présentent généralement sous la forme de lotion, huile, émulsion de type huile-dans-eau (H/E) ou eau-dans-huile (E/H), mousse, gel, stick ou spray. Elles contiennent, dans un support acceptable d'un point de vue cosmétique et à des concentrations diverses, un ou plusieurs filtres chimiques ou minéraux.

Les filtres chimiques sont des composés organiques qui absorbent la lumière en créant sur la peau une couche

filtrante qui neutralise les rayons UV. Ainsi lorsque la molécule de filtre solaire reçoit une radiation UV, elle passe dans un état excité en absorbant l'énergie du rayonnement puis retourne dans son état stable. Par ce mécanisme, la radiation lumineuse est convertie en chaleur et donc dissipée. Ainsi, les filtres chimiques agissent de la même façon que la mélanine, le pigment photoprotecteur naturel de la peau, dont la synthèse est stimulée par les UV-B.

Les associations de filtres, qui ont des spectres d'absorption différents, et les quantités mises en oeuvre, sont sélectionnées. Cette sélection se fait en fonction du niveau de protection solaire recherché et de la zone du corps à protéger, en tenant ainsi compte du degré de vulnérabilité dû à l'épaisseur de la peau.

Au cours des dernières décennies, des filtres chimiques de plus en plus performants ont été développés, présentant une photostabilité accrue et de larges spectres d'absorption vis-à-vis des UV-A et/ou UV-B. Ces filtres chimiques assurent une bonne photoprotection mais leur utilisation massive est de plus en plus décriée. En effet, ils possèdent souvent des propriétés allergisantes et peuvent être toxiques ou nuisibles s'ils pénètrent à travers la barrière épidermique. Ainsi, plusieurs filtres sont soupçonnés d'être des perturbateurs endocriniens. En outre, les filtres sont souvent peu biodégradables et deviennent des polluants environnementaux. Entre autres exemples d'effet écotoxique, les filtres solaires auraient un impact négatif sur les récifs marins et seraient notamment responsables du phénomène de blanchiment corallien (Danovaro *et al.* 2008).

Les filtres minéraux ou écrans physiques sont, quant à eux, des poudres inertes et opaques qui reflètent la lumière. Des exemples de filtres minéraux bien connus sont l'oxyde de zinc, l'oxyde de fer, le dioxyde de titane et l'oxyde de cérium. Les filtres minéraux agissent comme des miroirs et reflètent la radiation solaire. L'avantage de ces filtres est qu'ils ne pénètrent pas dans la peau, sont hypoallergéniques, et en général moins écotoxiques.

Toutefois, les compositions contenant des fortes doses de filtres minéraux laissent des traces blanches sur la peau, s'étalent difficilement et peuvent dessécher la peau. Ces problèmes ont été aujourd'hui résolus en réduisant les filtres minéraux à l'état de nanoparticules. Mais, le recul n'est pas encore suffisant pour pouvoir affirmer avec certitude que les écrans minéraux sous forme de nanoparticules n'ont aucune conséquence néfaste sur l'organisme humain. La tendance générale est donc de limiter, dans la mesure du possible, l'utilisation de filtres chimiques ou minéraux dans les produits solaires.

Une alternative ou un complément à la protection solaire externe est la protection solaire interne, qui consiste à renforcer les défenses de la peau en agissant sur les mécanismes endogènes qui régulent la réponse naturelle des cellules aux radiations solaires.

Pour lutter contre le stress photoxydatif et les dommages induits par les rayons UV, les cellules possèdent des systèmes de défense complexes. Ces mécanismes sont présents dans toutes les cellules de l'organisme et sont plus particulièrement développés au niveau des cellules de la peau, en raison de son exposition aux UV.

La protection interne a pour point de départ, l'observation selon laquelle ces défenses cellulaires endogènes peuvent être induites, afin d'améliorer l'efficacité de la réponse cutanée aux dégâts et mutations causés par la radiation solaire et/ou les polluants environnementaux.

Un médiateur clé de la réponse antioxydante de la peau est la protéine de la famille bZIP, Nrf2 *(« Nuclear factor erythroid-2-related factor 2* »). La protéine Nrf2, aussi connue sur le nom de NFE2L2, est un facteur de transcription qui contrôle l'expression de plusieurs gènes antioxydants au niveau des cellules. A l'état physiologique, la protéine Nrf2 est liée à la protéine Keap-1 (« *Kelch-like ECH-associated protein 1* »), formant un complexe cytoplasmique inhibant l'action de Nrf2. En réponse à un stress oxydatif et en présence des inducteurs de la réponse antioxydante, Keap-1 est inactivée permettant une libération de Nrf2 : Le facteur de transcription Nrf2 actif est transloqué dans le noyau où il se fixe à une classe de promoteurs spécifiques de l'expression génique, les ARE (« *Antioxidant Response Element* »). Il induit ainsi la transcription de nombreux gènes impliqués dans la réponse au stress oxydatif et dans la détoxification cellulaire.

Au niveau des cellules de la peau, Nrf2 est décrit pour son rôle central dans le processus de protection vis-à-vis des dommages induits par les UV, en activant l'expression de nombreux gènes impliqués dans la détoxification des ROS. Par ailleurs, la protéine Nrf2 stimule la production d'enzymes de détoxification de phase II, impliquées dans la synthèse d'antioxydants (notamment le glutathion, le NADPH et la thioredoxine) et la conjugaison permettant de greffer des radicaux moléculaires pour favoriser l'élimination de ces métabolites (Gorrini *et al.,* 2013).

En pratique, la fonction de la protéine Nrf2 a été mise en évidence en examinant le génotype d'une lignée de souris pour lesquelles l'expression du gène Nrf2 est supprimée (souris *Knock-out* (KO) *nrf-2-/-).* Ainsi, les souris KO Nrf2 montrent une réduction du niveau d'expression, basal et induit, des gènes cytoprotecteurs et sont plus vulnérables à la toxicité induite par les ROS (Copple *et al.,* 2008). En outre, les souris KO Nrf2 présentent un taux d'apoptose cellulaire et de dommages oxydatifs plus importants dans les kératinocytes après irradiation UV-B (Kawachi *et al.,* 2008).

Dans cette étude, une seule irradiation des souris KO Nrf2 avec des UV-B induit une réaction inflammatoire plus forte et plus longue que pour les souris sauvages pour ce gène (Kawachi *et al.,* 2008). A l'inverse, l'activation de la protéine Nrf2 protège efficacement les cellules murines des dommages des ROS *in vitro* et *in vivo.*

Par ailleurs, le facteur de transcription Nrf2 est constitutivement exprimé par les kératinocytes de la peau qui se trouve dans un état normal, et surexprimé durant le processus de cicatrisation (Braun *et al.,* 2002).

Des inducteurs de l'activation du facteur de transcription Nrf2 ont été identifiés, notamment le sulforaphane, un composé organosulfuré du groupe des isothiocyanates que l'on trouve dans les plantes crucifères comme le brocoli, le chou de Bruxelles ou le chou-fleur (Dinkova-Kostova *et al.,* 2006).

Dans des modèles de souris, il a été montré que l'application topique d'extraits de brocoli, contenant du sulforaphane, exerce un effet protecteur vis-à-vis de l'inflammation (Saw *et al.,* 2011) et de la photocarcinogenèse (Dinkova-Kostova *et al.,* 2006) UV-B-induites. Cependant, le sulphoraphane est une molécule très instable et n'est, à ce titre, pas adaptée au développement de produits solaires.

Il existe donc un besoin évident d'identifier de nouveaux actifs cosmétiques, formulables dans les produits solaires et capables d'agir en tant qu'inducteurs des réponses antioxydantes des cellules, notamment par le biais de l'activation de la voie Nrf2.

**Description de l'invention**

De façon surprenante, la Demanderesse a mis en évidence que des compositions dermocosmétiques solaires comprenant du trimethoxybenzyl acetylsinapate (INCI), un composé stable, répondent au besoin d'une composition permettant d'assurer une protection cellulaire endogène vis-à-vis des radiations UV. Notamment, cet actif possède des propriétés d'induction de l'expression génique contrôlée par le facteur de transcription Nrf2, sur lequel il exerce un effet comparable à celui de l'actif de référence, le sulphoraphane. De telles compositions cosmétiques ou dermatologiques présentent donc un intérêt évident dans la prévention des dégâts causés par les rayonnements UV, avantageusement en combinaison avec des filtres solaires dits classiques.

A noter que l'utilisation des dérivés de l'acide sinapinique a déjà été préconisée dans le domaine de la cosmétique ou de la pharmacie. En particulier, le document WO 2014/108629 décrit une longue liste de 41 dérivés, le trimethoxybenzyl acetylsinapate correspondant au composé 5. Celui-ci n'est décrit que pour son activité potentiellement amincissante, son effet anti-vieillissement via une compensation du déséquilibre de la production de protéines de la peau, et son activité blanchissante.

La désignation INCI trimethoxybenzyl acetylsinapate correspond à la molécule 3-[4-(acetyloxy)-3,5-dimethoxyphenyl] -(3,4,5-trimethoxyphenyl)methyl ester-(2E)-Propenoic acid (numéro CAS 1469299-98-6), caractérisée par la structure suivante :

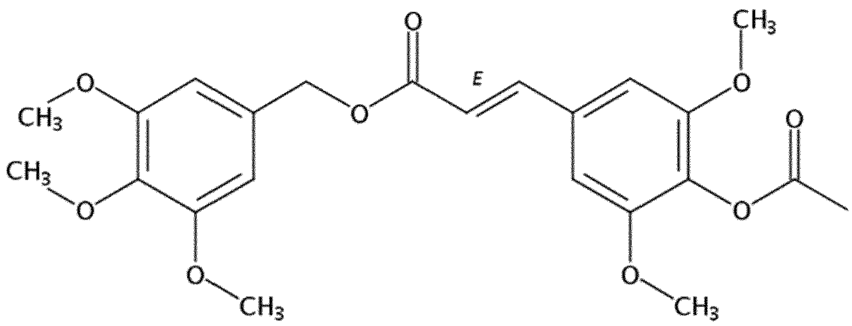

Par la suite, la désignation INCI sera utilisée pour dénommer cette molécule.

Le trimethoxybenzyl acetylsinapate est connu en qualité d'activateur de la synthèse de DKK-1 *(Dickkopf-1),* un messager intercellulaire impliqué dans la mélanogénèse (Yamaguchi *et al.,* 2009). DKK-1 active la voie de signalisation Wnt (*Winnt*) et permet ainsi de réduire les niveaux de synthèse de mélanine dans les mélanocytes. Le trimethoxybenzyl acetylsinapate a donc un effet dépigmentant médié par l'activation de DKK-1.

Cependant, à la connaissance de la Demanderesse, les propriétés antioxydantes et photoprotectrices du trimethoxybenzyl acetylsinapate, notamment grâce à sa fonction d'activateur de la voie Nrf-2 illustrée dans la présente demande, n'ont jamais été rapportées.

Selon un premier aspect, la présente invention concerne une composition cosmétique ou dermatologique comprenant du trimethoxybenzyl acetylsinapate. Selon un mode de réalisation privilégiée en rapport avec ses propriétés photoprotectrices, une telle composition comprend en outre au moins un filtre solaire, avantageusement un filtre UV.

Selon un autre aspect, la présente invention concerne l'utilisation du trimethoxybenzyl acetylsinapate dans une composition cosmétique ou dermatologique pour son activité antioxydante et photoprotectrice.

Avantageusement, le trimethoxybenzyl acetylsinapate représente entre 0,001% et 10% en poids total de la composition, de préférence entre 0,01% et 1%.

Toute forme de trimethoxybenzyl acetylsinapate peut être employée dans une composition au sens de l'invention. Le trimethoxybenzyl acetylsinapate peut être obtenu à partir d'extraits de plantes ou microorganismes ou encore

par synthèse chimique. Ainsi, la composition selon l'invention peut comprendre du trimethoxybenzyl acetylsinapate d'origine chimique ou d'origine naturelle, de préférence d'origine chimique.

En pratique, le trimethoxybenzyl acetylsinapate peut être utilisé dans la composition selon l'invention sous forme purifiée ou isolée, de pureté au moins égale à 60%, de préférence au moins égale à 70%, avantageusement au moins égale à 80%, encore plus avantageusement au moins égale à 90%, voire au moins égale à 95%.

Alternativement, il peut se présenter sous forme de mélange avec un support susceptible d'augmenter sa solubilité et/ou sa biodisponibilité cutanée.

Dans un mode de réalisation particulier, le principe actif trimethoxybenzyl acetylsinapate est vectorisé dans un support argileux. En pratique, la matière première INACLEAR®, commercialisée par les sociétés INABATA et PHARMASYNTHESE et correspondant aux désignations INCI Bentonite (and) Trimethoxybenzyl acetylsinapate, peut être utilisée dans le cadre de la présente invention.

Comme déjà dit et de manière avantageuse, la composition selon l'invention comprend, en outre, au moins un filtre UV.

Au sens de l'invention, le terme « filtre UV» englobe les composés organiques ou minéraux capables de filtrer les UV-A, les UV-B et/ou les UV-C.

Ainsi, l'adjonction de filtres UV dans une composition contenant du trimethoxybenzyl acetylsinapate permet d'améliorer l'effet de protection de la peau, des phanères, des lèvres, des cheveux et/ou des muqueuses, des effets néfastes des rayonnements UV en combinant une forme de protection externe à la protection interne mise en évidence dans la présente demande.

Selon l'invention, il peut aussi bien s'agir de filtres minéraux que de filtres chimiques ou organiques.

Les compositions selon l'invention peuvent contenir un ou plusieurs filtres UV à large spectre, c'est-à-dire des composés ou des mélanges qui absorbent les UV-A, les UV-B, les UV-C et éventuellement la lumière visible.

Parmi les filtres organiques à large spectre, les filtres correspondant aux désignations INCI suivantes peuvent être utilisés dans le cadre de l'invention : tris biphenyl triazine, bis ethylhexyloxyphenol methoxyphenyl triazine, methylene bis-benzotriazolyl tetramethylbutylphenol. A titre d'exemple, ceux-ci sont commercialisés par la société BASF sous les noms de TINOSORB S®/FINOSORB AQUA®, TINOSORB A2B®, TINOSORB M®, respectivement. Un autre exemple de filtre à large spectre adapté à la composition selon l'invention répond à la désignation INCI diethylhexyl butamido triazone, par exemple commercialisé par la société SIGMA 3V sous le nom d'UVASORB HEB®.

Ainsi et dans un mode de réalisation particulier, la composition selon l'invention comprend au moins un filtre choisi dans le groupe suivant de composés identifiés par leur désignation INCI : tris biphenyl triazine, bis ethylhexyloxyphenol methoxyphenyl triazine, et methylene bis-benzotriazolyl tetramethylbutylphenol, diethylhexyl butamido triazone, ou leurs mélanges.

Avantageusement, la composition comprend le filtre bis-ethylhexyloxyphenol methoxyphenyl triazine.

Selon un autre mode de réalisation, à la place ou en plus du ou des filtre(s) à large spectre, la composition contient au moins un filtre UV-A et/ou UV-B, organique et/ou minéral, qui peut se présenter en phase aqueuse (lipophile) et/ou huileuse (liposoluble).

Ainsi et à titre d'exemple, la composition selon l'invention peut contenir des filtres UV-B liposolubles, susceptibles de contribuer à la stabilisation ou à la solubilisation des filtres à large spectre ou encore de se stabiliser réciproquement et par ce fait, augmenter le facteur de protection solaire (FPS ou « *SPF* » en anglais).

Avantageusement, de tels filtres correspondent aux désignations INCI suivantes: homosalate, octocrylene, ethylhexyl salicylate, ethylhexyl triazone.

Dans un mode de réalisation préféré, la composition selon l'invention comprend de l'ethylhexyl triazone, commercialisé par BASF sous le nom d'UVINUL T150®.

Dans un autre mode de réalisation, le filtre UV-B liposoluble est 1'$\alpha$-(triméthylsilyl)-$\omega$-(triméthylsilyloxy)poly[oxy(diméthyl)silylène]-co-[oxy(méthyl)(2-{4-[2,2-bis(éthoxycarbonyl)vinyl]phénoxy}-1-méthylèneéthyl)silylène]-co-[oxy(méthyl)(2-(4-[2,2-bis(éthoxycarbonyl)vinyl]phénoxy)prop-1-ényl)silylène], un polymère siliconé capable de filtrer dans l'UV-B. Ce filtre correspond par exemple à la matière première cosmétique Parsol SLX®, commercialisée par la société DSM selon la désignation INCI polysilicone-15.

Dans un mode de réalisation préféré, la composition selon l'invention comprend au moins un filtre UV-B choisi dans le groupe suivant de composés identifiés par leur désignation INCI : homosalate, octocrylene, ethylhexyl salicylate, ethylhexyl triazone, polysilicone-15, ou leurs mélanges.

Dans un mode de réalisation particulier, la composition est exempte des filtres suivants : 4-methylbenzylidene camphor, benzophenone-2, benzophenone-3, ethylhexyl methoxycinnamate.

Dans un mode de réalisation avantageux, la composition selon l'invention comprend au moins un filtre UV-A, afin d'assurer une filtration complète de la partie nuisible du spectre solaire.

Des filtres UV-A avantageux au sens de la présente invention sont le butyl methoxydibenzoylmethane (INCI) et le diethylamino hydroxybenzoyl hexyl benzoate (INCI), correspondant respectivement aux matières premières Parsol 1789® commercialisée par la société DSM et UVINUU® A+ commercialisée par la société BASF.

Dans un mode de réalisation particulier, le filtre UV-A est le bis-(diethylaminohydroxybenzoyl benzoyl) piperazine (INCI) (numéro CAS 919803-06-8), correspondant par exemple à la matière première C1332® commercialisée par la société BASF.

Ainsi et dans un mode de réalisation préféré, la composition selon l'invention comprend au moins un filtre choisi dans le groupe suivant de composés identifiés par leur désignation INCI : butyl methoxydibenzoylmethane, diethylamino hydroxybenzoyl hexyl benzoate, bis-(diethylaminohydroxybenzoyl benzoyl) piperazine, ou leurs mélanges.

D'autres filtres UV avantageux au sens de la présente invention sont des filtres hydrosolubles, comme par exemple :

- le filtre correspondant à la désignation INCI disodium phenyl dibenzimidazole tetrasulfonate, notamment disponible sous la dénomination Neo Heliopan® AP (Symrise) ;
- le filtre correspondant à la désignation INCI phenylbenzimidazole sulfonic acid, notamment disponible sous la dénomination Neo Heliopan® hydro (Symrise), de préférence en combinaison avec un acide aminé basique, avantageusement de l'arginine. En pratique, l'acide aminé basique représente entre 0,5 et 2% en poids total de la composition, de préférence entre 1 et 1,5%.

Dans un mode de réalisation préféré, la composition selon l'invention comprend au moins un filtre hydrosoluble choisi dans le groupe suivant de composés identifiés par leur désignation INCI : Disodium phenyl dibenzimidazole tetrasulfonate, phenylbenzimidazole sulfonic acid, ou leurs mélanges.

Avantageusement, les filtres minéraux inorganiques, ou écrans minéraux, sont des oxydes métalliques et/ou d'autres composés difficilement solubles ou insolubles dans l'eau, en particulier les oxydes de titane ($TiO_2$), de zinc (ZnO), de fer ($Fe_2O_3$), de zirconium ($ZrO_2$), de silicium ($SiO_2$), de manganèse (par exemple MnO), d'aluminium ($Al2O_3$), ou de cérium ($Ce_2O_3$).

Selon un mode de réalisation particulier, les filtres minéraux inorganiques peuvent être utilisés sous forme de prédispersion huileuse ou aqueuse disponible sur le marché. Ces prédispersions peuvent être additionnées avantageusement d'auxiliaires de dispersion et/ou de médiateurs de solubilisation.

Les filtres minéraux inorganiques peuvent également être traités en surface ou encapsulés, afin de leur conférer un caractère hydrophile, amphiphile ou hydrophobe. Ce traitement de surface peut consister en ce que les filtres minéraux soient dotés d'une mince pellicule inorganique et/ou organique hydrophile et/ou hydrophobe.

Dans un mode de réalisation préféré, la composition selon l'invention comprend au moins un écran minéral choisi dans le groupe suivant de composés identifiés par leur désignation INCI : zinc oxide, titanium dioxide, ou leurs mélanges.

Les listes des filtres UV cités pouvant être mis en oeuvre au sens de la présente invention sont bien entendu donnés à titre indicatif et non limitatif.

De manière avantageuse, les filtres UV tels que décrits ci-dessus, présents dans la composition selon l'invention, représentent de 0,1 % à 30 % en poids total de la composition, avantageusement de 0,5 % à 20 %, encore plus avantageusement de 1 % à 15 %, afin de proposer des compositions cosmétiques qui protègent la peau ou les phanères de la totalité du domaine du rayonnement UV.

Selon un mode de réalisation particulier, la composition selon l'invention présente un facteur de protection solaire (« *SPF* » ou FPS) supérieur ou égal à 10, de préférence supérieur ou égal à 20, avantageusement supérieur ou égal à 30, encore plus avantageusement supérieur ou égal à 50.

Selon un mode de réalisation préféré, la composition selon l'invention comporte un ratio de protection UV-A/UV-B égal ou supérieur à 1/3.

La composition selon l'invention, constituée au minimum de trimethoxybenzyl acetylsinapate, avantageusement combiné à au moins un filtre UV, peut en outre comprendre un « booster » de SPF, c'est-à-dire un agent amplificateur du facteur de protection solaire, et/ou un photostabilisant, c'est à dire un ingrédient qui permet d'augmenter le SPF ou de photostabiliser les filtres, un tel ingrédient n'étant pas considéré lui-même comme un filtre solaire. On peut par exemple citer :

- le butyloctyl salicylate (INCI), photostabilisant représentant avantageusement entre 0,01 % et 10 % en poids total de la composition, encore plus avantageusement ente 0,1 % et 2 %. Cette matière première est par exemple commercialisée par la société HALLSTAR sous le nom de Hallbrite® BHB ;
- le benzotriazolyl dodecyl p-cresol (INCI), photostabilisant représentant avantageusement entre 0,01 % et 10 % en poids total de la composition, encore plus avantageusement entre 0,1 % et 2 %. Cette matière première est par exemple commercialisée par la société BASF sous le nom de TINOGARD® TL ;
- le pongamol (INCI), molécule végétale absorbant dans les UV-A, représentant avantageusement entre 0,5 et 2% en poids total de la composition, encore plus avantageusement de l'ordre de 1%. A titre d'exemple, la matière première Pongamia Extract commercialisée par la société GIVAUDAN peut être utilisée dans le cadre la présente

invention ;

- l'ethylhexyl methoxycrylene (INCI), photostabilisant, solubilisant et « booster » de SPF représentant avantageusement entre 1 % et 5 % en poids total de la composition. La matière première SolaStay® S1 commercialisée par la société HALLSTAR peut être utilisée dans le cadre de la présente invention ;
- un copolymère de styrène acrylate (INCI : styrene/acrylate copolymer), représentant de préférence entre 1% et 10% en poids total de la composition selon l'invention. Les matières premières SunSpheres® H53 et SunSpheres® PGL Polymer, commercialisées par la société DOW CHEMICALS, peuvent être utilisées dans le cadre de la présente invention ;
- le diethylhexyl syringylidene malonate (INCI), représentant avantageusement entre 1 % et 10 % en poids total de la composition. La matière première OXYNET® ST, commercialisée par la société MERCK, peut être utilisée dans le cadre de la présente invention ;
- un polyester hydrodispersible, correspondant aux désignations INCI polyester-5 (and) Sodium silicoaluminate, représentant avantageusement entre 1 % et 10 % en poids total de la composition, notamment l'EASTMANN AQ™38S Polymer commercialisé par la société SAFIC-ALCAN ;
- un copolymère d'acrylate ayant une température de transition vitreuse de -5 °C à -15 °C telle que mesurée par calorimétrie différentielle à balayage, ledit copolymère représentant avantageusement entre 1 % et 10 % en poids total de la composition. Par exemple, un polymère correspondant à la désignation INCI Acrylate copolymer, tel que la matière première EPITEX 66, commercialisée par la société DOW CHEMICALS, peut être utilisée dans le cadre de la présente invention.

Dans un mode de réalisation particulier, la composition selon l'invention comprend également une ou plusieurs substances aptes à filtrer la lumière visible, en particulier la lumière bleue. A titre d'exemple, les composés décrits dans le document EP 1 484 051 peuvent être employés pour assurer une filtration de la lumière bleue.

Selon un autre mode de réalisation, la composition selon l'invention peut comprendre en outre un extrait de la bactérie *Arthrobacter agilis*, notamment un extrait riche en caroténoïdes, tel que décrit dans le document WO 2014/167247. Avantageusement, la composition selon l'invention comprend de 0,00001 % à 0,1 % en poids total de la composition, encore plus avantageusement de 0,0001 % à 0,001 % d'un tel extrait sec.

Dans un mode de réalisation privilégié, la composition selon l'invention comprend en outre d'autres composants pouvant contribuer à la protection interne par une action qui peut consister en une protection de l'ADN, une diminution de l'immunosuppression induite par les radiations UV, une action antiradicalaire ou un effet combiné de ces actions.

L'action protectrice d'une préparation selon l'invention contre le stress oxydatif ou à l'encontre de l'effet des radicaux libres peut être encore améliorée si celle-ci comprend en outre un ou plusieurs antioxydants, aisément sélectionnés par l'homme du métier, par exemple dans la liste suivante : le totarol, le magnolol, l'honokiol, les acides aminés et leurs dérivés, les imidazoles (acide urocanique) et leurs dérivés, des peptides (D et/ou L-carnosine) et leurs dérivés (par exemple l'ansérine, l'hypotaurine, la taurine), les caroténoïdes, les carotènes ($\alpha$-carotène, $\beta$-carotène, lycopène) et leurs dérivés, l'acide chlorogénique et ses dérivés, l'acide lipoïque et ses dérivés (acide dihydrolipoïque), l'aurothioglucose, le propylthiouracile et autres thiols (thiorédoxine, glutathion, cystéine, cystine, cystamine et leurs esters glycosyle, N-acétyle, méthyle, éthyle, propyle, amyle, butyle et lauryle, palmitoyle, oléyle, $\gamma$-linoléique, cholestéryle et glycéryle) ainsi que des sels de ceux-ci, le thiodipropionate de dilauryle, le thiodipropionate de distéaryle, l'acide thiodipropionique et ses dérivés, les composés sulfoximine (sulfoximine de buthionine, l'homocystéine sulfoximine, les buthionine sulfones, penta-, hexa- et heptathionine sulfoximine), des agents chélatants (tels que les acides $\alpha$-hydroxygras, l'acide palmitique, l'acide phytique, la lactoferrine), les $\alpha$-hydroxyacides (tels que l'acide citrique, lactique, ou malique), l'acide humique, l'acide biliaire, les extraits de bile, la bilirubine, la biliverdine, l'EDTA, l'EGTA, le tétraméthylène phosphonate pentasodique d'éthylènediamine et ses dérivés, les acides gras insaturés et leurs dérivés, la vitamine A et ses dérivés (palmitate de vitamine A), le benzoate de coniféryle de la résine de benjoin, l'acide rutinique et ses dérivés, l'$\alpha$-glycosyl rutine, l'acide férulique et ses dérivés, le furfurylideneglucitol, la carnosine, le butylhydroxytoluène, le butylhydroxyanisole, l'acide nordihydroguaiarétique, trihydroxybutyrophenone, la quercétine, l'acide urique et ses dérivés, le mannose et les dérivés de ceux-ci, le zinc et ses dérivés (ZnO, $ZnSO_4$), le sélénium et ses dérivés (sélénométhionine), les stilbènes et leurs dérivés (l'oxyde de stilbène, l'oxyde trans-stilbène).

Dans un mode de réalisation particulier, la composition selon l'invention contient en outre de l'acide glycyrrhétinique, un dérivé ou un sel de cet acide, utilisé comme apaisant (agent anti-inflammatoire) et représentant entre 0,01 % et 2 % en poids total de la composition, de préférence entre 0,1 % et 1 %.

Selon une autre caractéristique privilégiée de l'invention, la composition cosmétique et/ou dermatologique contient au moins un, voire tous les constituants suivants exerçant une activité biologique *in vivo* sur les cellules de la peau, des lèvres, des cheveux et/ou des muqueuses soumises à une rayonnement UV-A et/ou UV-B, respectivement :

- un agent antiradicalaire préservant les structures cellulaires, tel que par exemple la vitamine E et/ou ses dérivés liposolubles ou hydrosolubles, en particulier le tocotriénol et/ou le tocophérol, représentant avantageusement entre

0,001 et 10 % en poids total de la composition, encore plus avantageusement entre 0,02 et 2 %, de préférence de l'ordre de 0,04 % ;

- un agent limitant l'immunosuppression, tel que par exemple la vitamine PP, représentant avantageusement entre 0,001 et 1 % en poids total de la composition, encore plus avantageusement de 0,01 % à 0,3 %;
- un agent protecteur de la protéine p53, tel que par exemple l'épigallocatéchine gallate (EGCG), représentant avantageusement entre 0,001 et 0,1 % en poids total de la composition, encore plus avantageusement de 0,005 % à 0,05 %.

La composition selon l'invention peut également comprendre en outre des extraits peptidiques de soja et/ou de blé, tels que ceux décrits dans EP 2 059 230.

En pratique, les extraits peptidiques proviennent de graines de soja et de blé sont issu d'une hydrolyse enzymatique desdites graines par l'intermédiaire de peptidases qui permet de récupérer des peptides d'une taille moyenne de 700 Daltons. Préférentiellement, l'extrait peptidique de soja est l'extrait identifié sous le numéro CAS 68607-88-5, de même que l'extrait peptidique de blé est l'extrait identifié sous le numéro CAS 70084-87-6. Les extraits de blé et soja peuvent correspondre aux désignations INCI Hydrolyzed wheat protein et Hydrolyzed soy protein, respectivement.

Dans un mode de réalisation particulier, les extraits peptidiques de soja et de blé sont utilisés ensemble, par exemple dans un rapport pondéral respectivement compris entre 80/20 et 20/80, avantageusement compris entre 70/30 et 30/70, de préférence égal à 60/40.

Dans un mode de réalisation avantageux, les extraits peptidiques de soja et/ou de blé sont exempts de tripeptides synthétiques GHK (glycyl-histidyl-lysine ; INCI : Tripeptide-1). En pratique, les extraits peptidiques de soja et/ou blé représentant de 0,01 à 20 % en poids total de la composition, avantageusement de 0,1 % à 10 %, encore plus avantageusement de 0,2 % à 0,7 %.

Dans un mode de réalisation alternatif, la composition selon l'invention comprend, conformément aux enseignements du document FR 2 865 398, l'association d'au moins un acide aminé choisi dans le groupe constitué par l'ectoïne, la créatine, l'ergothionéine et/ou la carnosine, ou leurs sels physiologiquement acceptables, et du mannitol ou un dérivé du mannitol.

De préférence, la composition selon l'invention comprend dans un milieu physiologiquement acceptable l'acide aminé ou l'un de ses sels, seul ou en mélange dans des proportions comprises entre 0,001 % et 10 % en poids total de la composition, et de préférence entre 0,01 % et 5 %.

La composition selon la présente invention comprend, de préférence, dans un milieu physiologiquement acceptable, du mannitol ou l'un de ses dérivés, dans des proportions comprises entre 0,01% et 30 % en poids total de la composition, avantageusement entre 0,1 % et 10 %.

Dans un mode de réalisation privilégié, la composition selon l'invention comprend de l'ectoïne et du mannitol.

Selon un mode de réalisation particulier, la composition selon l'invention contient un ou plusieurs autres agents bronzants ou autobronzants. Il peut s'agir d'un autobronzant qui réagit avec les acides aminés de la peau selon une réaction de Maillard ou par l'intermédiaire d'une addition de Michael, ou bien un promoteur de la mélanogénèse ou un composé propigmentant qui favorise le bronzage naturel de la peau.

Une telle substance est de préférence présente dans la composition en quantité allant de 0,01 % à 20 % en poids total de la composition, avantageusement de 0,5 % à 1 5%, encore plus avantageusement de 1 % à 8 %.

Les substances autobronzantes peuvent être le 1,3-dihydroxyacétone (DHA), le glycérolaldéhyde, l'hydroxyméthylglyoxal, l'$\gamma$-dialdéhyde, l'érythrulose, le 6-aldo-D-fructose, la ninhydrine, la 5-hydroxy-1,4-naphtoquinone (juglone), la 2-hydroxy-1,4-naphtoquinone (lawsone) ou leurs combinaisons.

Les substances propigmentantes peuvent être l'hormone stimulant les mélanocytes ($\alpha$-MSH), des analogues peptidiques de l'$\alpha$-MSH, des agonistes du récepteur à l'endothéline-1, des agonistes des récepteurs $\mu$ opiacés, des agents stimulateurs d'AMPc, des agents stimulateurs de tyrosinase.

Dans un mode de réalisation particulier, la composition selon l'invention comprend en outre, en qualité d'autobronzant, une combinaison de dihydroxy méthylchromonyl palmitate et une forme lipophile de la tyrosine.

Comme décrit dans le document FR 3 031 301, cette association de principes actifs permet de stimuler efficacement le bronzage. Le dihydroxy méthylchromonyl palmitate (Numéro CAS : 1387636-35-2) correspond par exemple à l'ingrédient cosmétique commercialisé par la société MERCK sous le nom de RonaCare® Bronzyl. Elle se présente sous la forme d'une poudre blanche, sans odeur, avec une pureté supérieure à 99 %.

Selon un mode de réalisation particulier, le dihydroxy méthylchromonyl palmitate est présent dans la composition selon l'invention à hauteur de 0,01 % à 10 % en poids total de la composition, avantageusement de 0,05 % à 10 %, encore plus avantageusement de 0,1 % à 5 %, plus particulièrement de 0,1 % à 0,5 %.

La forme lipophile de la tyrosine, au sens de l'invention, est un ingrédient à base de tyrosine et présentant un caractère lipophile plus prononcé que la tyrosine. La forme lipophile de tyrosine peut notamment correspondre à l'oléoyl tyrosine (Numéro CAS : 147732-57-8), qui se retrouve, par exemple, dans l'ingrédient cosmétique liquide TYR-OL, commercialisé par la société SEDERMA, et qui comprend environ 50 % en poids d'oléoyl tyrosine dans du butylène

glycol (environ 30 % + environ 20 % d'acide oléique), ou bien dans l'ingrédient cosmétique liquide TYR-EXCEL, commercialisé par la société SEDERMA, qui comprend environ 50 % en poids d'oléoyl tyrosine, environ 20 % d'acide oléique (N° CAS : 112-80-1) et environ 30 % d'huile de *Luffa cylindrica* (huile de pépins de courge éponge; N° CAS 1242417-48-6).

Selon un autre mode de réalisation, la forme lipophile de la tyrosine correspond à une huile végétale dans laquelle a été formulée la tyrosine.

Selon un mode de réalisation particulier, l'huile végétale est de l'huile de tournesol oléique, avantageusement désodorisée. Ainsi, la matière première OLEOACTIF TYROSINE BASE HELIANTHUS ANNUS commercialisée par l'entreprise OLEOS, et correspondant aux désignations INCI *Helianthus annuus* seed oil (and) tyrosine (and) glyceryl stéarate, peut être utilisée dans le cadre de la présente invention.

En pratique, la forme lipophile de la tyrosine, telle que dans les ingrédients cosmétiques à base d'oléoyl-tyrosine (avantageusement à 50 % en poids) ou de la tyrosine formulée dans de l'huile végétale, représente entre 0,1 % et 10 % en poids total de la composition, avantageusement entre 1 et 3%, encore plus avantageusement entre 1 % et 1,5 %.

La composition selon l'invention peut également contenir des actifs ayant des propriétés dépigmentantes, comme par exemple :

- de l'azéilate de lysine, ou d'autres dérivés ou sels de l'acide azélaïque ;
- de l'andrographolide, notamment l'extrait *d'Andrographis paniculata* correspondant à la désignation INCI *Andrographis paniculata* leaf extract ;
- de l'acide ascorbique natif (vitamine C) ou ses dérivés, notamment les dérivés correspondant aux INCI Ascorbyl Glucoside Ethyl ascorbic acid, Ascorbyl methylsilanol pectinate, Sodium ascorbyl phosphate et Ascorbyl tetraisopalmitate ;
- de l'arbutine ou un extrait végétal la contenant, notamment l'extrait de busserole correspondant à la désignation INCI *Arctostaphylos uva-ursi* leaf extract ;
- de la glabridine ou un extrait végétal la contenant, notamment les extraits de réglisse correspondant à la désignation INCI *Glycyrrhiza glabra* root extract, *Glycyrrhiza inflata* root extract, *Glycyrrhiza uralensis* root extract ;
- les peptides biomimétiques correspondant aux désignations INCI hexapeptide 2 et/ou nonapeptide-1 ;
- un extrait aqueux d'une algue dénommée *Palmaria palmata,* notamment l'extrait correspondant à la désignation INCI *Palmaria palmata* extract ;
- le 4-n-butylresorcinol ;
- de la vitamine PP, également appelée niacinamide ou nicotinamide, et ses dérivés ;

ou leurs mélanges.

La composition selon l'invention peut également contenir des actifs ayant des propriétés cicatrisantes comme par exemple un agent antimicrobien choisi parmi les actifs correspondant aux désignations INCI suivantes : Copper sulfate, zinc sulfate, sodium hyaluronate, *Vitis vinifera* (grape) vine extract, ou leurs mélanges.

La composition selon l'invention peut également contenir des actifs ayant des propriétés sébocorrectices, kératolytiques, séboregulatrices et/ou une activité antiacnéique, afin de permettre la formulation de produits solaires traitant l'acné.

Par exemple, la composition selon l'invention peut comprendre un agent antimicrobien choisi parmi les actifs correspondant aux désignations INCI propyl gallate, dodecyl gallate, *Ginkgo biloba* leaf extract, bakuchiol, dihydromyricetine, zinc gluconate, salicylic acid, ou leurs mélanges.

La composition selon l'invention peut donc en outre comprendre au moins un ingrédient choisi dans la liste suivante :

- un agent amplificateur du facteur de protection solaire ou *booster SPF* ;
- un photostabilisant ;
- un extrait de la bactérie *Arthrobacter agilis*, avantageusement riche en caroténoïdes ;
- un méroterpène, avantageusement du bakuchiol ;
- au moins un polyol choisi parmi le xylitol, le rhamnose, le sorbitol et le mannitol ;
- un extrait de *Gingko biloba* ;
- du butyl hydroxytoluène (BHT) ;
- un extrait de la plante *Andrographis paniculata* ;
- un extrait de l'algue *Palmaria palmata* ;
- un extrait des algues *Laminaria ochroleuca, Blidingia Minima* ou *Laminaria Saccharina* ;
- un extrait de la plante *Zanthoxylum alatum* ;
- du panthénol ;
- un agent antiradicalaire préservant les structures cellulaires, tel que par exemple la vitamine E et/ou un de ses dérivés liposoluble ou hydrosoluble, en particulier le tocotriénol et/ou le tocophérol ;

- de l'acide salicylique ou un de ses dérivés ;
- un extrait de bois de cade ;
- un extrait de Boldo ;
- un extrait de Reine des prés ;
- de l'acide glycyrrhétinique ou un de ses dérivés, ou un de leurs sels ;
- un agent limitant l'immunosuppression, avantageusement la vitamine PP ;
- un agent protecteur de la protéine p53, avantageusement l'épigallocathéchine gallate (EGCG) ;
- un extrait d'huile de karanja issue du *Pongamia glabra* ;
- des paraffines linéaires ;
- de l'ATP (adénosine-5 tri-phosphate) ou un précurseur d'ATP, par exemple la $Gp_4G$ (diguanosine tétraphosphate), ou l'$Ap_4A$ (diadénosine tétraphosphate) ;
- un agent limitant l'action des ions fer impliqués dans la formation des radicaux libres, avantageusement l'EDTA ;
- un extrait peptidique de soja et/ou de blé ;
- un acide aminé ou dérivé d'acide aminé choisi parmi le groupe constitué de l'ectoïne, la créatine, l'ergothionéine, la carnosine, la tyrosine, la décarboxycamosine, la glutamine et leurs sels ;
- un agent bronzant ou autobronzant ;
- un agent dépigmentant ;
- un agent cicatrisant ;
- un agent sébocorrecteur, kératolytique, séborégulateur et/ou antiacnéique.

La composition selon l'invention peut également contenir des adjuvants comme ceux habituellement utilisés dans le domaine de la cosmétique, tels que des actifs, des conservateurs, des antioxydants, des agents complexants, des solvants, des parfums, des charges, des bactéricides, des électrolytes, des absorbeurs d'odeur, des matières colorantes ou encore des vésicules lipidiques. Le choix de ces adjuvants, ainsi que leurs concentrations, doivent être déterminés de telle sorte qu'ils ne modifient pas les propriétés et les avantages recherchés pour la composition de la présente invention.

La composition de l'invention est destinée à une application topique et plus particulièrement à une application sur la peau, les lèvres, les cheveux et/ou les muqueuses. Ainsi et dans le cadre de l'invention, une telle composition est notamment destinée à la protection de la peau et/ou des phanères, en particulier les muqueuses, les lèvres et les cheveux, contre le rayonnement UV.

La composition de l'invention peut se présenter sous toutes les formes galéniques normalement utilisées dans les domaines cosmétique et dermatologique, comme par exemple, mais de façon non limitative, sous la forme d'une solution aqueuse éventuellement gélifiée, d'une dispersion du type lotion, d'une émulsion Huile dans Eau (H/E) Eau dans Huile (E/H), plus ou moins fluide, ou d'une émulsion multiple comme par exemple une émulsion triple (E/H/E ou H/E/H), ou encore sous la forme d'une dispersion vésiculaire de type ionique (liposomes) et/ou non ionique, d'une composition biphasée dépourvue d'émulsionnants et gélifiants dont les phases immiscibles se séparent pendant le stockage, de mousse, de stick, d'huile anhydre, de spray ou de brume.

Dans un mode de réalisation préféré, la composition selon l'invention est une émulsion E/H. Avantageusement, les émulsions E/H selon l'invention comprennent le PEG-30 dipolyhydroxystearate (INCI) en qualité d'émulsionnant.

De préférence, les émulsions E/H selon l'invention comprennent un solubilisant choisi dans le groupe suivant de composés identifiés par leur désignation INCI : dicaprylyl carbonate, C12-15 alkyl benzoate, dibutyl adipate, seuls ou en mélange. Ces émulsionnants et solubilisants sont disponibles sur le marché auprès de plusieurs fournisseurs.

Dans un mode de réalisation alternatif, la composition selon l'invention est une émulsion H/E. Avantageusement, les émulsions H/E selon l'invention comprennent un émulsionnant choisi dans le groupe suivant de composés identifiés par leur désignation INCI : potassium cetyl phosphate, glyceryl stéarate, PEG-100 stéarate et C20-22 alkyl phosphate/C20-C22 alkyl alcool, tribehenin PEG-20 Esters et leurs mélanges.

De préférence, les émulsions H/E selon l'invention comprennent un solubilisant choisi dans le groupe suivant de composés identifiés par leur désignation INCI : dicaprylyl carbonate C12-15 alkyl benzoate, dibutyl adipate, disopropyl sebacate seuls ou leurs mélanges. Ces émulsionnants et solubilisants sont disponibles sur le marché auprès de plusieurs fournisseurs.

La composition selon l'invention peut également comprendre des conservateurs. Tout conservateur apte à être utilisé dans des compositions cosmétiques ou dermatologiques peut être employé dans la formulation d'une composition selon l'invention.

Avantageusement, les conservateurs utilisés sont des alcanediols, encore plus avantageusement des 1,2-alcanediols ou 1,3-alcanediols, et leurs mélanges.

Dans un mode de réalisation préféré, le conservateur est un 1,2-diol choisi parmi le 1,2-pentanediol, le 1,2-hexanediol, le 1,2-heptanediol, le 1,2-octanediol et le 1,2-decanediol, ou leurs mélanges.

Dans un mode de réalisation alternatif, le conservateur est un 1,3-diol choisi parmi le 1,3-propanediol et le 1,3-butanediol, ou leurs mélanges. En pratique, ces alcanediols sont commercialisés par plusieurs entreprises, notamment la société SYMRISE ou MINACARE.

De préférence, la composition selon l'invention comprend entre 0,01 % et 2 % en poids total de la composition de diols, avantageusement entre 0,1 % et 1 %, par exemple 0,5 %.

Une autre contribution de l'invention concerne la mise en évidence de l'activité antioxydante d'une composition comprenant du trimethoxybenzyl acetylsinapate, via l'activation de Nrf2. Comme décrit dans la littérature, cette activité antioxydante est corrélée à une activité anti-radicalaire et un effet protecteur vis-à-vis du rayonnement UV.

Selon un autre aspect, la présente invention concerne donc une composition comprenant du trimethoxybenzyl acetylsinapate, avantageusement comme décrite ci-dessus notamment en combinaison avec un filtre UV, pour son utilisation comme :

- agent antioxydant ;
- agent anti-radicalaire ;
- agent de protection contre la pollution ;
- agent de protection contre les rayonnements UV.

Il en résulte qu'une telle composition peut être utilisée dans la prévention et/ou le traitement des altérations des cellules de la peau, des lèvres, des cheveux, et/ou des muqueuses causées par les radiations solaires, en particulier les rayonnements UV. A ce titre elle permet donc de lutter contre le vieillissement, en particulier le photovieillissement.

La manière dont l'invention peut être réalisée et les avantages qui en découlent ressortiront mieux des exemples de réalisation qui suivent, donnés à titre indicatif et non limitatif, à l'appui de la figure annexée.

**Figure 1** : Effet du trimethoxybenzyl acetylsinapate sur l'induction de la voie Nrf2 dans le modèle cellulaire « ARE Reporter-HepG2 ». Le pourcentage d'activation de Nrf2 dans les cellules traitées (lignée HepG2) après 18 heures de contact avec la molécule trimethoxybenzyl acetylsinapate est indiqué.

Exemples de réalisation

Les pourcentages indiqués sont donnés en poids de produit par rapport au poids total de la composition dans les tableaux ci-dessous.

**Exemple I - brume solaire SPF50+**

| **Nom INCI** | **%** |
|---|---|
| Alcohol denat. | 35,00 |
| Bis-ethylhexyloxyphenol methoxyphenyl triazine | 10,00 |
| Dibutyl adipate | 10,00 |
| Diethylamino hydroxybenzoyl hexyl benzoate | 10,00 |
| Homosalate | 10,00 |
| Diisopropyl sebacate | 5,00 |
| Ethylhexyl salicylate | 5,00 |
| Ethylhexyl triazone | 5,00 |
| Dicaprylyl carbonate | 4,9985 |
| Dicaprylyl ether | 3,09904 |
| Aqua/water/eau | 1,50 |
| Fragrance (parfum) | 0,30 |
| Trimethoxybenzyl acetylsinapate | 0,10 |
| Tocopherol | 0,002460 |

**Exemple II - crème H/E SPF30+**

| Nom INCI | % |
|---|---|
| Aqua/water/eau | 64,658 |
| Octocrylene | 7,05 |
| C12-15 alkyl benzoate | 5,00 |
| Methylene bis-benzotriazolyl tetramethylbutylphenol [nano] | 5,00 |
| Dicaprylyl carbonate | 4,9985 |
| Ethylhexyl methoxycinnamate | 2,9979 |
| Glycerin | 2,00 |
| Cetearyl alcohol | 1,30 |
| Isononyl isononanoate | 1,00 |
| Phenoxyethanol | 1,00 |
| Microcrystalline cellulose | 0,88 |
| Decyl glucoside | 0,75 |
| Coco-glucoside | 0,70 |
| Hydroxyethyl acrylate/sodium acryloyldimethyl taurate copolymer | 0,592 |
| C20-22 alkyl phosphate | 0,55 |
| C20-22 alcohols | 0,45 |
| Squalane | 0,408 |
| Disodium EDTA | 0,20 |
| Trimethoxybenzyl acetylsinapate | 0,15 |
| Cellulose gum | 0,12 |
| Polysorbate 60 | 0,088 |
| Propylene glycol | 0,04 |
| Sorbitan isostearate | 0,024 |
| Citric acid | 0,02 |
| Xanthan gum | 0,02 |
| BHT | 0,0021 |
| Tocopherol | 0,0015 |

**Exemple III - crème E/H SPF50+**

| Nom INCI | % |
|---|---|
| Aqua/water/eau | 45,057 |
| C12-15 alkyl benzoate | 24,90 |
| Bis-ethylhexyloxyphenol methoxyphenyl triazine | 5,00 |
| Butyl methoxydibenzoylmethane | 5,00 |

(suite)

| Nom INCI | % |
|---|---|
| Octocrylene | 5,00 |
| Ethylhexyl methoxycinnamate | 4,9965 |
| PEG-30 dipolyhydroxystearate | 3,00 |
| Glycerin | 2,00 |
| Dicaprylyl carbonate | 1,7392 |
| Tris-biphenyl triazine | 1,50 |
| Phenoxyethanol | 1,00 |
| Decyl glucoside | 0,225 |
| Disodium EDTA | 0,20 |
| Stearalkonium hectorite | 0,20 |
| Propylene carbonate | 0,060 |
| Trimethoxybenzyl acetylsinapate | 0,10 |
| Propylene glycol | 0,012 |
| Xanthan gum | 0,006 |
| BHT | 0,0035 |
| Tocopherol | 0,0008 |

**Exemple IV - Evaluation de l'induction de la voie Nrf2 par l'actif selon l'invention**

Le but de l'étude est d'évaluer l'induction de la voie Nrf2 dans le modèle cellulaire « ARE Reporter-HepG2 » (HepG2 ; BPS BIOSCIENCE) [Lee *et al.,* (2004), Dinkova-Kostova *et al.,* (2002)] par l'actif visé dans l'invention.

**IV.1 PROTOCOLE EXPERIMENTAL**

IV .1.1- Décongélation et amplification des cellules «ARE Reporter-HepG2»

La décongélation des cellules HepG2 transfectées a été réalisée selon les recommandations du fournisseur de cellules (BPS Bioscience).

Les cellules d'une ampoule ont été décongelées rapidement dans un bain-marie à 37 °C, puis transférées dans un tube contenant 10 ml de milieu MEM (Milieu Essentiel Minimum) supplémenté avec 10 % de SVF (Sérum de Veau Foetal), 1 % de Pénicilline/Streptomycine, 1 mM de pyruvate de sodium, 1% d'acides aminés non essentiels (= milieu M1). Après centrifugation 5 minutes à 200 g, les cellules du culot sont mises en suspension dans 7 ml de milieu de culture préchauffé et introduites dans un flacon de culture T25 $cm^2$ pour croissance.

Avant la confluence, les cellules sont détachées du support par incubation avec le réactif trypsine/EDTA (acide éthylène diamine tétra-acétique), puis centrifugées 5 minutes à 200 g. Les cellules du culot sont mises en suspension dans 5 ml de milieu de culture M1 contenant de la généticine 600 $\mu$g/mL (= milieu M2), puis elles sont ensemencées dans des flacons de culture T75 $cm^2$ pour amplification (15 ml de milieu M2).

Toutes les étapes de culture (amplification et incubation) ont été réalisées à 37°C, sous 5 % de $CO_2$.

IV.1.2- Préparation de l'actif à tester

La solution à tester a été préparée le jour de l'essai d'activation. La dilution a été réalisée dans du milieu M 1.

| Actif | Référence / N° lot | Solution mère | Doses testées |
|---|---|---|---|
| **Trimethoxybenzyl Acetylsinapate _INABATA** | LB189/696 | 1,25 % dans DMSO | **0,00063 %**<br>**0,00125 %** |

IV.1.3- Préparation du produit de référence

Une solution mère de sulforaphane (10 mM) (Sigma-Aldrich réf. S4441 - lot SLBR6147V) activateur de référence, a été préparée dans du DMSO, puis diluée en milieu M1 (concentration finale 3 μM).

IV.1.4- Détection de la cytotoxicité potentielle de la molécule à tester

*IV.1.4.1. Ensemencement des cellules*

Les cellules HepG2 ont été ensemencées, en milieu M1, dans des puits de plaques à 96 puits, à raison de 40 000 cellules/puits (volume de 100 μL). Seuls les 60 puits centraux des plaques ont été utilisés, les puits restants n'ont reçu que du PBS.

*IV.1.4.2. Traitement des cellules*

Après adhésion des cellules, le milieu de culture a été enlevé. Les cellules ont été traitées avec les produits à l'essai dilués dans le milieu de culture M1, pendant 18 heures (volume final : 50 μL). Sur chaque plaque, des puits « cellules témoins » ont été traités avec le milieu M1 contenant le même pourcentage de solvant DMSO que les puits traités avec les molécules à tester.

Après 18 heures d'incubation, les milieux ont été enlevés. Les cellules ont été rincées 1 fois avec 100 μl de PBS, puis traitées avec 100 μl de PBS contenant 0,5 mg/mL de MTT (bromure de 3-(4,5-dimethylthiazol-2-yl)-2,5-diphenyl tetrazolium).

Après 2h30 d'incubation à 37°C sous 5 % de $CO_2$, le MTT a été éliminé et les cellules ont été lysées par ajout de 100μL de DMSO.

*IV.1.4.3. Evaluation de la cytotoxicité*

La densité optique du bleu de formazan (MTT réduit par les déshydrogénases mitochondriales) a été mesurée par spectrophotométrie à 540 nm. Cette densité optique est directement proportionnelle au nombre de cellules métaboliquement actives.

*IV.1.4.4. Traitement des données*

Les résultats ont été exprimés en pourcentage de la densité optique des puits traités par rapport à la moyenne de la densité optique du groupe « cellules témoins » traitées avec le même pourcentage du même solvant.

Les moyennes et les écarts types ont été calculés.

IV.1.5- Evaluation de l'activation de Nrf2

*IV.1.5.1. Ensemencement des cellules*

En parallèle du test de cytotoxicité, des cellules HepG2 ont été également ensemencées dans des puits de plaques à 96 puits (plaques opaques avec fond transparent) à raison de 40 000 cellules/puits (volume de 100 μL), en milieu M1.

Seuls les 60 puits centraux des plaques ont été utilisés, les puits restants n'ont reçu que du PBS.

*IV.1.5.2. Traitement des cellules*

Après adhésion des cellules, le milieu de culture M1 a été enlevé. Les cellules ont été traitées (volume final: 50 μL) avec le produit à l'essai, dilué dans le milieu de culture M1 (n=3/concentration à tester, 1 concentration/produit), pendant 18 heures.

Sur chaque plaque, des puits « sans cellules » et des puits « cellules témoins solvant » ont été traités avec le milieu M1 contenant 0,5 % de DMSO.

Les puits « sans cellules » ont servi à la détection de la luminescence liée au bruit de fond du milieu. Les puits « cellules témoins » ont servi à la détection de la luminescence intrinsèque du système d'essais (sans activation).

Le sulforaphane à 3 μM a été testé comme activateur de référence.

*IV.1.5.3. Evaluation des effets*

A la fin de la période d'incubation (18 heures à 37°C, 5 % de $CO_2$), les cellules ont été lysées par ajout de 100 µL du réactif « *One-step luciferase assay system* ». La luminescence émise par la luciférase néo-synthétisée a été évaluée avec un luminomètre Optima.

*IV.1.5.4. Traitement des données*

Le traitement des données a été réalisé selon le protocole suivant :

- Soustraction de la valeur moyenne de la luminescence des puits « sans cellules » à toutes les valeurs de luminescence obtenues dans les puits non traités (« cellules témoins ») et traités.
- Moyenne de la luminescence des puits « cellules témoins »
- Le pourcentage d'induction de la luciférase (proportionnel au niveau d'activation de Nrf2 par les molécules testées) a été calculé selon la formule :

$$\frac{100 \times (\text{Luminescence des puits traités avec les molécules})}{\text{Moyenne de la luminescence des « cellules témoins solvant »}}$$

Les moyennes et les écarts types des pourcentages d'activation ont été calculés.

**IV.2. RESULTATS ET CONCLUSIONS**

Aucune des doses retenues de la composition selon l'invention n'induit une cytotoxicité significative dans le modèle cellulaire étudié.

Le sulforaphane, testé à 3 µM, augmente la luminescence émise par les cellules HepG2 traitées, en comparaison à la luminescence émise par les cellules traitées avec le témoin solvant correspondant, à hauteur de 438 %. Cette augmentation valide le test.

L'actiftrimethoxybenzyl acetylsinapate présente un taux d'activation de Nrf2 dose-dépendant, avec un taux d'activation à 164 % à la dose de 0,00125 % par rapport au témoin solvant correspondant (figure 1).

**BIBLIOGRAPHIE**

Braun S., Hanselmann C., Gassmann M.G., Auf dem Keller U., Born-Berclaz C., Chan K., Kan Y.W., Werner S. (2002) Nrf2 transcription factor, a novel target ofkeratinocyte growth factor action which regulates gène expression and inflammation in the healing skin wound. Mol Cell Biol. 22(15):5492-5505.

Copple I.M., Goldring C.E., Kitteringham N.R., Park B.K. (2008) The Nrf2-Keap1 defence pathway: rôle in protection against drug-induced toxicity. Toxicology. 246(1):24-33.

Danovaro R., Bongiomi L., Corinaldesi C., Giovannelli D., Damiani E., Astolfi P., Greci L. Pusceddu A. (2008) Sunscreens cause coral bleaching by promoting viral infections. Environ Health Perspect 116(4):441-447.

Dinkova-Kostova, AT. Holtzclaw WD, Cole, R.N. , Itoh, K. , Wakabayashi, N. , Katoh, Y. , Yamamoto, M., Talalay. P. (2002). Direct évidence that sulfhydryl groups of Keap1 are the sensors regulating induction of phase 2 enzymes that protect against carcinogens and oxidants. Proc Natl Acad Sci USA. 99(18): 11908-11913.

Dinkova-Kostova A.T., Jenkins S.N., Fahey J.W., Ye L., Wehage S.L., Liby K.T., Stephenson K.K. Wade K.L., Talalay P. (2006) Protection against UV-light-induced skin carcinogenesis in SKH-1 high-risk mice by sulforaphane-containing broccoli sprout extracts. Cancer Lett. 240(2):243-252.

Gorrini C., Baniasadi P.S., Harris I.S., Silvester J., Inoue S., Snow B., Joshi P.A., Wakeham A., Molyneux S.D., Martin B., Bouwman P., Cescon D.W., Elia A.J., Winterton-Perks Z., Cruickshank J., Brenner D., Tseng A., Musgrave M., Berman H.K., Khokha R., Jonkers J., Mak T.W., Gauthier M.L. (2013) BRCA1 interacts with Nrf2 to regulate antioxidant signaling and cell survival. J Exp Med. 210(8):1529-1544.

Kawachi Y., Xu X., Taguchi S., Sakurai H., Nakamura Y., Ishii Y., Fujisawa Y., Furuta J., Takahashi T., Itoh K., Yamamoto M., Yamazaki F., Otsuka F. (2008) Atténuation of UVB-induced sunbum reaction and oxidative DNA damage with no altérations in UVB-induced skin carcinogenesis in Nrf2 gene-deficient mice. J Invest Dermatol. 128(7): 1773-1779.

Lee, J.M. et. Johnson J.A. (2004) An important rôle of Nrf2-ARE pathway in the cellular défense mechanism. J Biochem Mol Biol. 37(2): 139-143.

Lloyd, S.A. (1993) Stratospheric ozone depletion. Lancet. 342(8880): 1156-8.

Saw C.L., Huang M.T., Liu Y., Khor T.O., Conney A.H., Kong A.N. (2011) Impact of Nrf2 on UVB-induced skin inflammation/photoprotection and photoprotective effect of sulforaphane. Mol Carcinog. 2011 50(6):479-486.
Yamaguchi Y., Passeron T., Hoashi T.,Watabe H., Rouzaud F., Yasumoto K., Hara T., Tohyama C., Katayama I., Miki T.,Hearing V.J.Dicckopf 1 (2009) DKK1 regulates skin pigmentation and thickness by affecting Wnt/beta-catenin signaling in keratinocytes. FASEB J. (22) 1009-1020.

## Revendications

1. Composition comprenant du trimethoxybenzyl acetylsinapate et au moins un filtre UV.

2. Composition selon la revendication 1, ***caractérisée* en ce que** le trimethoxybenzyl acetylsinapate représente entre 0,001 % et 10 % en poids total de la composition, avantageusement entre 0,01 % et 1 %.

3. Composition selon l'une des revendications 1 ou 2, ***caractérisée* en ce que** le filtre est un filtre UV-A.

4. Composition selon la revendication 3, ***caractérisée* en ce que** le filtre est choisi dans le groupe suivant : butyl methoxydibenzoylmethane, diethylamino hydroxybenzoyl hexyl benzoate, bis-(diethylaminohydroxybenzoyl benzoyl) piperazine, et leurs mélanges.

5. Composition selon l'une des revendications précédentes, ***caractérisée* en ce que** le filtre est un filtre à large spectre.

6. Composition selon la revendication 5, ***caractérisée* en ce que** le filtre à large spectre est choisi dans le groupe suivant : tris biphenyl triazine, bis ethylhexyloxyphenol methoxyphenyl triazine, méthylène bis-benzotriazolyl tetramethylbutylphenol, diethylhexyl butamido triazone et leurs mélanges, avantageusement le bis ethylhexyloxyphenol methoxyphenyl triazine.

7. Composition selon l'une des revendications 1 ou 2, ***caractérisée* en ce qu'**elle comprend au moins un filtre UV-A, avantageusement tel que défini par la revendication 4, et au moins un filtre à large spectre, avantageusement tel que défini par la revendication 6.

8. Composition selon l'une des revendications précédentes, ***caractérisée* en ce que** le filtre UV représente de 0,1 % à 30 % en poids total de la composition, avantageusement de 0,5 % à 20 %, encore plus avantageusement de 1 % à 15 %.

9. Composition selon l'une des revendications précédentes, ***caractérisée* en ce qu'**elle comprend en outre un ingrédient choisi dans le groupe suivant :

   - un agent amplificateur du facteur de protection solaire ;
   - un agent photostabilisant ;
   - un agent apte à filtrer la lumière visible, en particulier la lumière bleue ;
   - un extrait de la bactérie *Arthrobacter agilis,* avantageusement riche en caroténoïdes ;
   - au moins un polyol choisi parmi le xylitol, le rhamnose, le sorbitol et le mannitol ;
   - un extrait des algues *Laminaria ochroleuca, Blidingia minima* ou *Laminaria saccharina ;*
   - un extrait de la plante *Zanthoxylum alatum ;*
   - du panthénol ;
   - de la vitamine E ou un de ses dérivés hydrophile ou lipophile, ou un de leurs sels, avantageusement le tocotriénol ou le tocophérol ;
   - de l'acide salicylique ou un de ses dérivés ;
   - un extrait de bois de cade ;
   - un extrait de Boldo ;
   - un extrait de Reine des prés ;
   - de l'acide glycyrrhétinique ou un de ses dérivés ou sels ;
   - un agent limitant l'immunosuppression, avantageusement la vitamine PP,
   - un agent protecteur de la protéine p53, avantageusement l'épigallocathéchine gallate (EGCG) ;
   - un extrait d'huile de karanja issue du *Pongamia glabra ;*
   - des paraffines linéaires ;
   - de l'ATP (adénosine-5 tri-phosphate) ou un précurseur d'ATP, par exemple la $Gp_4G$ (diguanosine tétraphos-

phate), ou l'$Ap_4A$ (diadénosine tétraphosphate) ;
- un agent limitant l'action des ions fer impliqués dans la formation des radicaux libres, avantageusement l'EDTA ;
- un extrait peptidique de soja et/ou de blé ;
- un acide aminé ou dérivé d'acide aminé choisi dans le groupe constitué de l'ectoïne, la créatine, l'ergothionéine, la carnosine, la tyrosine, la décarboxycamosine, la glutamine et leurs sels ;
- un agent bronzant ou autobronzant ;
- un agent dépigmentant ;
- un agent cicatrisant ;
- un agent sébocorrecteur, kératolytique, séborégulateur et/ou antiacnéique.

**10.** Composition selon l'une des revendications précédentes, ***caractérisée* en ce qu'**elle comprend du mannitol et de l'ectoïne.

**11.** Composition selon l'une des revendications précédentes, ***caractérisée* en ce qu'**elle se présente sous la forme d'une crème, d'une lotion, d'une solution, d'une émulsion, d'un gel, d'une huile, d'un stick, d'une mousse, d'un spray ou d'une brume.

**12.** Composition comprenant du trimethoxybenzyl acetylsinapate pour utilisation comme agent antioxydant.

**13.** Composition comprenant du trimethoxybenzyl acetylsinapate pour utilisation dans le traitement des altérations des cellules de la peau, des lèvres, des cheveux, et/ou des muqueuses causées par les radiations solaires, en particulier les rayonnements UV.

**14.** Composition pour son utilisation selon la revendication 12 ou 13, ***caractérisée* en ce que** la composition est telle que définie dans les revendications 1 à 11.

**15.** Composition pour son utilisation selon l'une des revendications 12 à 14, ***caractérisée* en ce que** la composition est appliquée sur la peau, les lèvres, les cheveux, et/ou les muqueuses.

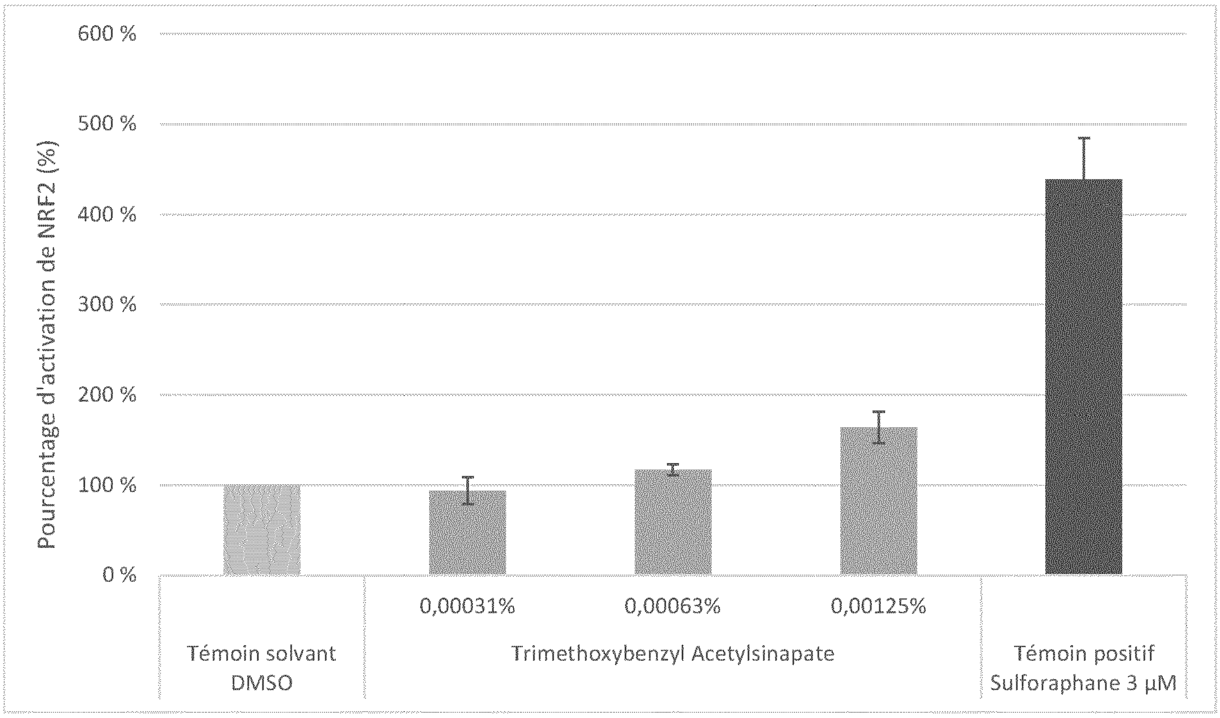
600 %
500 %
400 %
300 %
200 %
100 %
0 %
Pourcentage d'activation de NRF2 (%)
0,00031%
0,00063%
0,00125%
Témoin solvant DMSO
Trimethoxybenzyl Acetylsinapate
Témoin positif Sulforaphane 3 µM

**Figure 1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2014108629 A
- EP 1484051 A
- WO 2014167247 A
- EP 2059230 A
- FR 2865398
- FR 3031301


### Littérature non-brevet citée dans la description


- *CHEMICAL ABSTRACTS,* 1469299-98-6
- *CHEMICAL ABSTRACTS,* 919803-06-8
- *CHEMICAL ABSTRACTS,* 68607-88-5
- *CHEMICAL ABSTRACTS,* 70084-87-6
- *CHEMICAL ABSTRACTS,* 1387636-35-2
- *CHEMICAL ABSTRACTS,* 147732-57-8
- *CHEMICAL ABSTRACTS,* 112-80-1
- *CHEMICAL ABSTRACTS,* 1242417-48-6
- **BRAUN S. ; HANSELMANN C. ; GASSMANN M.G. ; AUF DEM KELLER U. ; BORN-BERCLAZ C. ; CHAN K. ; KAN Y.W. ; WERNER S.** Nrf2 transcription factor, a novel target ofkeratinocyte growth factor action which regulates gène expression and inflammation in the healing skin wound. *Mol Cell Biol,* 2002, vol. 22 (15), 5492-5505
- **COPPLE I.M. ; GOLDRING C.E. ; KITTERINGHAM N.R. ; PARK B.K.** The Nrf2-Keap1 defence pathway: rôle in protection against drug-induced toxicity. *Toxicology.,* 2008, vol. 246 (1), 24-33
- **DANOVARO R. ; BONGIOMI L. ; CORINALDESI C. ; GIOVANNELLI D. ; DAMIANI E. ; ASTOLFI P. ; GRECI L. ; PUSCEDDU A.** Sunscreens cause coral bleaching by promoting viral infections. *Environ Health Perspect,* 2008, vol. 116 (4), 441-447
- **DINKOVA-KOSTOVA, AT ; HOLTZCLAW WD ; COLE, R.N. ; ITOH, K. ; WAKABAYASHI, N. ; KATOH, Y. ; YAMAMOTO, M. ; TALALAY. P.** Direct évidence that sulfhydryl groups of Keap1 are the sensors regulating induction of phase 2 enzymes that protect against carcinogens and oxidants. *Proc Natl Acad Sci USA.,* 2002, vol. 99 (18), 11908-11913
- **DINKOVA-KOSTOVA A.T. ; JENKINS S.N. ; FAHEY J.W. ; YE L. ; WEHAGE S.L. ; LIBY K.T. ; STEPHENSON K.K. ; WADE K.L. ; TALALAY P.** Protection against UV-light-induced skin carcinogenesis in SKH-1 high-risk mice by sulforaphane-containing broccoli sprout extracts. *Cancer Lett.,* 2006, vol. 240 (2), 243-252
- **GORRINI C. ; BANIASADI P.S. ; HARRIS I.S. ; SILVESTER J. ; INOUE S. ; SNOW B. ; JOSHI P.A. ; WAKEHAM A. ; MOLYNEUX S.D. ; MARTIN B.** BRCA1 interacts with Nrf2 to regulate antioxidant signaling and cell survival. *J Exp Med.,* 2013, vol. 210 (8), 1529-1544
- **KAWACHI Y. ; XU X. ; TAGUCHI S. ; SAKURAI H. ; NAKAMURA Y. ; ISHII Y. ; FUJISAWA Y. ; FURUTA J. ; TAKAHASHI T. ; ITOH K.** Atténuation of UVB-induced sunbum reaction and oxidative DNA damage with no altérations in UVB-induced skin carcinogenesis in Nrf2 gene-deficient mice. *J Invest Dermatol,* 2008, vol. 128 (7), 1773-1779
- **LEE, J.M. ; JOHNSON J.A.** An important rôle of Nrf2-ARE pathway in the cellular défense mechanism. *J Biochem Mol Biol,* 2004, vol. 37 (2), 139-143
- **LLOYD, S.A.** Stratospheric ozone depletion. *Lancet,* 1993, vol. 342 (8880), 1156-8
- **SAW C.L. ; HUANG M.T. ; LIU Y. ; KHOR T.O. ; CONNEY A.H. ; KONG A.N.** Impact of Nrf2 on UVB-induced skin inflammation/photoprotection and photoprotective effect of sulforaphane. *Mol Carcinog,* 2011, vol. 50 (6), 479-486
- **YAMAGUCHI Y. ; PASSERON T. ; HOASHI T. ; WATABE H. ; ROUZAUD F. ; YASUMOTO K. ; HARA T. ; TOHYAMA C. ; KATAYAMA I. ; MIKI T.** DKK1 regulates skin pigmentation and thickness by affecting Wnt/beta-catenin signaling in keratinocytes. *FASEB J.,* 2009, vol. 22, 1009-1020